# EUROPÄISCHE PATENTSCHRIFT

(11) **EP 2 184 601 B1**
(45) Veröffentlichungstag und Bekanntmachung des Hinweises auf die Patenterteilung: **05.08.2015**
(21) Anmeldenummer: 09014062.5
(22) Anmeldetag: 10.11.2009
(51) Int. Cl.: G01N 27/407, G01N 33/00

(54) **Sensorvorrichtung zum Messen einer Ammoniakkonzentration**
Sensor device for measuring an ammoniac concentration
Dispositif de capteur destiné à mesurer une concentration d'ammoniaque

(30) Priorität: 11.11.2008 DE 102008056791
(43) Veröffentlichungstag der Anmeldung: 12.05.2010
(73) Patentinhaber: Volkswagen Aktiengesellschaft, 38440 Wolfsburg (DE)
(72) Erfinder: Daetz, Michael, 38473 Tiddische (DE); Hemmi, Martin, 38518 Gifhorn (DE); Müller, Oliver, 38473 Tiddische (DE)

(56) Entgegenhaltungen:
- EP-A1- 1 640 578
- WO-A1-2005/098410
- DE-A1-102005 042 490
- US-A1- 2006 108 222
- US-A1- 2008 190 768

## Beschreibung

Die Erfindung betrifft eine Sensorvörrichtung zum Messen einer Gaskonzentration in einem Abgasstrom eines Verbrennungsmotors, mit einem für Stickoxide (NOₓ) und Ammoniak (NH₃) empfindlichen Sensor und einem Gehäuse in dem der Sensor aufgenommen ist, eine mit der Sensorvorrichtung versehene Abgasreinigungsvorrichtung sowie ein Verfahren zum Herstellen der Sensorvorrichtung.

Sensorvorrichtung zum Messen einer Stickoxidkonzentration und einer Ammoniakkonzentration sind bekannt. Sie können beispielsweise in Kraftfahrzeugen mit einem Dieselmotor und einer Abgasnachbehandlung mittels einer selektiven katalytischen Reduktion von Stickoxiden (SCR-System) zur Steuerung und Dosierung eines Reaktionsmittels eingesetzt werden. insbesondere sind Sensoren bekannt, die nur zur Messung einer Stickoxid (NOₓ) Konzentration ausgelegt sind. Dabei kann es zu Querempfindlichkeiten gegenüber Ammoniak kommen. Die EP 1 640 578 A1 beschreibt beispielsweise ein Verfahren zum Betreiben einer Brennkraftmaschine und Vorrichtung zur Durchführung des Verfahrens, wobei ein NOₓ-Sensor eine Querempfindlichkeit gegenüber einem Reagenzmittel aufweist. Die DE 10 2007 029 154 A1 offenbart einen Mischpotentialsensor mit beschleunigtem Ansprechverhalten zur Detektion verschiedener Gase in Gasgemischen, sowie Verfahren zum Betrieb desselben. Die DE 10 2006 008 227 A1 betrifft einen Gassensor zur Bestimmung einer wasserstoffhaltigen Komponente und/oder ihrer Konzentration in einem Messgas. Die DE 10 2005 012 568 A1 offenbart eine Vorrichtung zur Entfernung von Stickoxiden aus Brennkraftmaschinenabgas und ein Verfahren zur Dosierung eines Zuschlagstoffs für Brennkraftmaschinenabgas. Die Vorrichtung weist einen ersten Katalysatorteil und einen zweiten Katalysatorteil auf, denen Abgassensoren, die als Stickoxidsensoren ausgeführt sind, nachgeschaltet sind, wobei zumindest einer der Sensoren auch als Ammoniaksensor ausgeführt sein kann.

Aus der DE 10 2005 042 490 A1 ist eine Sensorvörrichtung zum Messen zumindest einer Gaskonzentration am Abgasstrom eines Verbrennungsmotors bekannt, mit einem für Stickoxide und Ammoniak empfindlichen Sensor.

Ferner ist aus der WO 2005/098410 A1 ein Sensor bekannt, mit einem Schutzgehäuse um dem Sensor nicht direkt dem Abgasstrom und den darin enthalten Verunreinigungen wie Ruß auszusetzten. Das Schutzgehäuse umgibt dabei das Sensorelement und umfasst darüber hinaus eine Gaskontaktfläche bzw. eine Innenwand mit einer Beschichtung mit Trockenmittel zur Aufnahme von Wasserdampf aus dem Gasstrom sowie Edelmetallen zur katalytischen umsetzung von Schadstoffen im Abgas.

Aus der US 2006/108222 A1 ist bereits ein Gassensor, beispielsweise für Sauerstoff oder NOx bekannt, der zur Messung eines Luftkraftstoffverhältnisses in einer Mischung verwendet wird die einem automotive Verbrennungsmotor zugeführt wird. Der Gassensor ist mit einem Sensorelement und einer Abdeckung versehen. Die Abdeckung weist eine Beschichtung mit einem hydrophilen Film auf, mit einem Wasserkontaktwinkel von weniger als 70°. Damit wird eine minimale Exposition des Sensorelements für Wasser erreicht ohne, dass die Response des Sensors verschlechtert wird.

Aufgabe der Erfindung ist es, einen Stickoxidsensor zu ermöglichen, der gegenüber Ammoniak eine reproduzierbare und langzeitstabile Empfindlichkeit aufweist, der insbesondere für einen Einsatz bei einer Abgasnachbehandlung eines Abgases eines mit einer mageren Gemischzusammensetzung betreibbaren Verbrennungsmotors eines Kraftfahrzeuges geeignet ist.

Die Aufgabe ist mit den Merkmalen der unabhängigen Ansprüche gelöst.

Die Aufgabe ist bei einer Sensorvorrichtung zum Messen einer Gaskonzentration in einem Abgasstrom eines Verbrennungsmotors, mit einem für Stickoxide (NOx) und Ammoniak (NH3) empfindlichen Sensor und einem Gehäuse in dem der Sensor aufgenommen ist dadurch gelöst, dass die dem Sensor benachbarte Innenfläche des Gehäuses gegenüber Ammoniak im Wesentlichen katalytisch inaktiv ist oder mit einer Behandlung in einen gegenüber Ammoniak im Wesentlichen katalytisch inaktiven Zustand versetzt ist, derart, dass keine oder zumindest nur eine minimale Beeinflussung eines mittels des Sensors ermittelbaren Messsignals durch das Gehäuse stattfindet.

Der Sensor der Sensorvorrichtung ist innerhalb des Gehäuses so angeordnet, dass er mit dem Abgasstrom oder zumindest einem Teilstrom bzw. Nebenstrom des Abgasstroms in Berührung kommt. Die dem Sensor benachbart angeordnete Innenfläche des Gehäuses wird ebenfalls mit dem Abgasstrom beaufschlagt. Vorteilhaft weist diese jedoch gegenüber dem in dem Abgasstrom vorhandenen Ammoniak im Wesentlichen keine katalytische Aktivität auf. Vorteilhaft findet also keine oder zumindest nur eine minimale Beeinflussung eines mittels des Sensors der Sensorvorrichtung ermittelbaren Messsignals durch das Gehäuses der Sensorvorrichtung statt. Bei der Sensorvorrichtung kann es sich vom prinzipiellen Aufbau her beispielsweise um einen Stickoxid-Sensor handeln, der auch eine Empfindlichkeit gegenüber Ammoniak aufweist. Erfindungsgemäß ist vorteilhaft zusätzlich die Behandlung des Gehäuses vorgesehen.

Das Gehäuse kann ein den eigentlichen Sensor umgebendes Schutzrohr umfassen. Vorteilhaft tritt im Wesentlichen keine Änderung der Empfindlichkeit gegenüber Ammoniak auf, da im Wesentlichen keine katalytische Reaktion des möglicherweise in dem Abgasstrom vorhandenen Ammoniaks in dem Schutzrohr der Sensorvorrichtung stattfindet. Vorteilhaft ist dies auch der Fall, falls die Geschwindigkeit des Abgasstroms innerhalb des Gehäuses bzw. Schutzrohrs der Sensorvorrichtung sehr klein ist. Trotz einer sich dadurch ergebenden großen Verweildauer von Ammoniakmolekülen an der Innenfläche kann mittels der Behandlung eine Reaktion von Ammoniak (NH₃) zu Stickstoffdioxid (N₂) und Wasser (H₂O) verhindert werden bzw. auf einem Minimum reduziert werden. Das mittels der Sensorvorrichtung ermittelbare Sensorsignal weist daher eine stabile Empfindlichkeit gegenüber dem Ammoniak auf. Unter stabiler Empfindlichkeit kann eine im Wesentlichen gleiche Empfindlichkeit gegenüber dem Ammoniak bei unterschiedlichen Umgebungsbedingungen verstanden werden. Bei den Umgebungsbedingungen kann es sich beispielsweise um eine Temperatur und/oder eine Strömungsgeschwindigkeit des Abgasstroms handeln. Die Umgebungsbedingungen können von unterschiedlichen Betriebsbedingungen bzw. Lastzuständen des Verbrennungsmotors abhängen. Vorteilhaft kann ein mittels der Sensorvorrichtung ermittelbares Messsignal für eine stabile Steuerung einer Dosierung eines Reduktionsmittels, beispielsweise einer Harnstofflösung, in den Abgasstrom dienen. Dabei können bestmöglich Ammoniakdurchbrüche erkannt und mittels der Regelung und/oder Steuerung verhindert werden. Vorteilhaft kann mittels des verbesserten Messsignals der Sensorvorrichtung eine instabilere und/oder kürzere Zeitkonstanten aufweisende Strecke geregelt werden. Dies bedeutet vorteilhaft, dass beispielsweise kleinere Katalysatoren, insbesondere SCR-Katalysatoren für die Abgasnachbehandlung einsetzbar sind.

Bei einem Ausführungsbeispiel der Sensorvorrichtung ist vorgesehen, dass das Gehäuse ein Metall aufweist. Metalle können unbehandelt eine katalytische Aktivität gegenüber Ammoniakmolekülen aufweisen. Mittels der Behandlung ist es möglich, diese auszuschließen oder zumindest auf ein Minimum zu reduzieren. Vorteilhaft weisen Metallgehäuse die notwendige chemische, temperatur- und/oder mechanische Stabilität auf, um den Sensor ausreichend zu schützen.

Bei einem weiteren Ausführungsbeispiel der Sensorvorrichtung ist vorgesehen, dass die Behandlung eine Oxidschicht des Metalls aufweist. Vorteilhaft kann mittels der Oxidschicht eine katalytische Aktivität des Metalls reduziert werden, bestenfalls gänzlich ausgeschlossen werden.

Bei einem weiteren Ausführungsbeispiel der Sensorvorrichtung ist vorgesehen, dass die Oxidschicht gegen Fettgaskomponenten und Ammoniak chemisch stabil ist. Fettgaskomponenten und Ammoniak können reduzierend wirken. Vorteilhaft kann die Oxidschicht so ausgebildet sein, dass trotzt der Anwesenheit dieser Reduktionsmittel diese sich nicht verändert, also ihre katalytische Vorgänge verhindernde Eigenschaft beibehält.

Bei einem weiteren Ausführungsbeispiel der Sensorvorrichtung ist vorgesehen, dass die Oxidschicht mittels Tempern des Gehäuses bei hohen Temperaturen, insbesondere oberhalb von 800°C, und Aussetzen einer sauerstoffhaltigen Atmosphäre herstellbar ist. Vorteilhaft kann die Oxidschicht durch ein einfaches Tempern des Gehäuses bei Anwesenheit der sauerstoffhaltigen Atmosphäre hergestellt werden.

Bei einem weiteren Ausführungsbeispiel der Sensorvorrichtung ist vorgesehen, dass die Oxidschicht mittels beaufschlagen mit Salpetersäure, insbesondere konzentrierter Salpetersäure, herstellbar ist. Vorteilhaft kann die Oxidschicht mittels einer einfachen Nassbehandlung mittels der Salpetersäure hergestellt werden.

Bei einem weiteren Ausführungsbeispiel der Sensorvorrichtung ist vorgesehen, dass - wie an sich bekannt - das Metall des Gehäuses einen austenitischen Edelstahl aufweist. Austenitische Edelstähle können eine gute chemische Beständigkeit aufweisen und lassen sich leicht und kostengünstig zu dem Gehäuse verarbeiten.

Bei einem weiteren Ausführungsbeispiel der Sensorvorrichtung ist vorgesehen, dass das Metall des Gehäuses eine Nickel (Ni)-Basis-Legierung aufweist. Nickel-Basis-Legierungen sind ebenfalls leicht zu handhaben und können kostengünstig in ein Gehäuse umgeformt werden, beispielsweise mittels spanabhebender Bearbeitung, wie Drehen und/oder Bohren.

Bei einem weiteren Ausführungsbeispiel der Sensorvorrichtung ist vorgesehen, dass die Behandlung mittels Beifügen eines Katalysatorgiftes herstellbar ist. Vorteilhaft kann das Katalysatorgift ebenfalls die katalytische Aktivität der inneren Fläche herabsetzen oder bestenfalls auf Null reduzieren.

Bei einem weiteren Ausführungsbeispiel der Sensorvorrichtung ist vorgesehen, dass das Katalysatorgift Schwefel aufweist. Schwefel weist gute katalytische Reaktionen verhindernde Eigenschaften auf und ist leicht beifügbär.

Bei einem weiteren Ausführungsbeispiel der Sensorvorrichtung ist vorgesehen, dass das Katalysatorgift mittels Diffusion beifügbar ist. Die Diffusion kann beispielsweise zunächst mittels Bedampfen mit Schwefel induziert werden. Gegebenenfalls kann zusätzlich noch ein Tempern eingesetzt werden.

Die Aufgabe ist außerdem bei einer Abgasreinigungsvorrichtung zur Reinigung eines Abgasstroms mittels einer selektiven katalytischen Reduktion eines Verbrennungsmotors eines Kraftfahrzeuges, mit einem in den Abgasstrom geschalteten SCR-Katalysator, einer dem SCR-Katalysator vorgeschalteten Dosiervorrichtung zum Dosieren eines Reaktionsmittels in den Abgasstrom, einer dem SCR-Katalysator und der Dosiervorrichtung zugeordneten Sensorvorrichtung dadurch gelöst, dass die Sensorvorrichtung wie vorab beschrieben ausgebildet ist. Es ergeben sich die vorab beschriebenen Vorteile: Insbesondere kann die Dosierung des Reduktionsmittels, beispielsweise eine wässrige Harnstofflösung, genauer und präziser erfolgen, wobei unerwünschte Ammoniakdurchbrüche besser gemessen beziehungsweise erkannt und daher besser vermieden werden können.

Bei einem Ausführungsbeispiel der Abgasreinigungsvorrichtung ist vorgesehen, dass der SCR-Katalysator ein Volumen von weniger als 6 Liter, insbesondere weniger als 4 öder 3 Liter, vorzugsweise ca. 2 Liter, aufweist. Vorteilhaft kann mittels der Sensorvorrichtung ein vorteilhaft deutlich genaueres Signal zur Detektierung und Verhinderung von Ammoniakdurchbrüchen generiert werden, so dass vorteilhaft der SCR-Katalysator vergleichsweise klein ausgelegt werden kann. Insbesondere ist eine genauere Dosierung und/oder eine genauere Gemischbildung des Verbrennungsmotors möglich, was insgesamt zu niedrigeren Emissionen und einer kleiner auslegbaren Abgasreinigungsvorrichtung beiträgt.

Die Aufgabe ist außerdem bei einem Verfahren zum Herstellen einer vorab beschriebenen Sensorvorrichtung durch ein Versehen der Innenfläche des Gehäuses mit einer Behandlung, die gegenüber dem Ammoniak im Wesentlichen keine katalytische Aktivität aufweist, gelöst. Mittels des Verfahrens ist die Herstellung der vorab beschriebenen vorteilhaften Sensorvorrichtung möglich. Insofern ergeben sich die vorab beschriebenen Vorteile.

Bei einer Ausführungsform des Verfahrens ist zumindest ein Schritt der folgenden Gruppe vorgesehen: Oxidieren der Innenfläche, Oxidieren der Innenfläche mittels Tempern der Innenfläche in Anwesenheit einer sauerstoffhaltigen Atmosphäre, Oxidieren der Innenfläche mittels Nassbehandeln, Nassbehandeln der Innenfläche mittels Salpetersäure, insbesondere konzentrierter Salpetersäure, Beifügen eines Katalysatorgifts zu der Innenfläche, insbesondere mittels Dotieren, insbesondere mittels Diffusion, insbesondere mittels Ionenimplantation, und Beifügen des Katalysatorgifts in Form von Schwefel. Die Innenfläche kann auf einfache Art und Weise mittels Oxidieren katalytisch deaktiviert werden. Die Oxidation der Oberfläche bildet die Behandlung bzw. eine Schutzschicht, die wirkungsvoll einen Kontakt des Abgasstroms, insbesondere des darin mitgeführten Ammoniaks, mit dem eigentlichen Metall des Gehäuses der Sensorvorrichtung verhindert. Die Oxidation kann auf einfache Art und Weise mittels Tempern in Anwesenheit der sauerstoffhaltigen Atmosphäre oder mittels der Nassbehandlung, insbesondere mittels der Salpetersäure, insbesondere in konzentrierter Form, erfolgen. Zusätzlich oder alternativ ist es möglich, die Innenfläche mit dem Katalysatorgift zu versehen, was ebenfalls zu einer katalytischen Deaktivierung der Innenfläche führt. Das Katalysatorgift, beispielsweise in Form von Schwefel, kann auf einfache Art und Weise mittels Dotieren, insbesondere Diffusion und/oder Ionenimplantation, der Innenfläche beigefügt werden. Alternativ oder zusätzlich ist es auch möglich, das Katalysatorgift insgesamt dem Material des Gehäuses, das die Innenfläche aufweist, beizufügen, beispielsweise direkt bei einem Herstellungsprozess des Metalls.

Weitere Vorteile, Merkmale und Einzelheiten ergeben sich aus der nachfolgenden Beschreibung, in der unter Bezug auf die Zeichnung ein Ausführungsbeispiel im Einzelnen beschrieben ist. Gleiche, ähnliche und/oder funktionsgleiche Teile sind mit gleichen Bezugszeichen versehen. Es zeigen:
- Figur 1: eine Abgasreinigungsvorrichtung zur Reinigung eines Abgasstroms mittels einer selektiven katalytischen Reduktion eines Verbrennungsmotors eines Kraftfahrzeuges;
- Figur 2: eine Sensorvorrichtung zum Messen zumindest einer Gaskonzentration in einem Abgasstrom;
- Figur 3: eine schematische Darstellung eines Längsschnitts eines Teils einer Sensorvorrichtung mit einem einen Sensor umgebenden Gehäuse;
- Figur 4: eine Ansicht eines Details einer Innenfläche des in Figur 3 dargestellten Gehäuses, wobei die Innenfläche eine Behandlung aufweist;
- Figur 5: das in Figur 4 gezeigte Detail, wobei die Behandlung ein Katalysatorgift aufweist, dass im Bereich der Innenfläche eine höhere Konzentration aufweist; und
- Figur 6: das in Figur 5 gezeigte Detail, wobei das Katalysatorgift in einem Material des Gehäuses gleich verteilt ist.

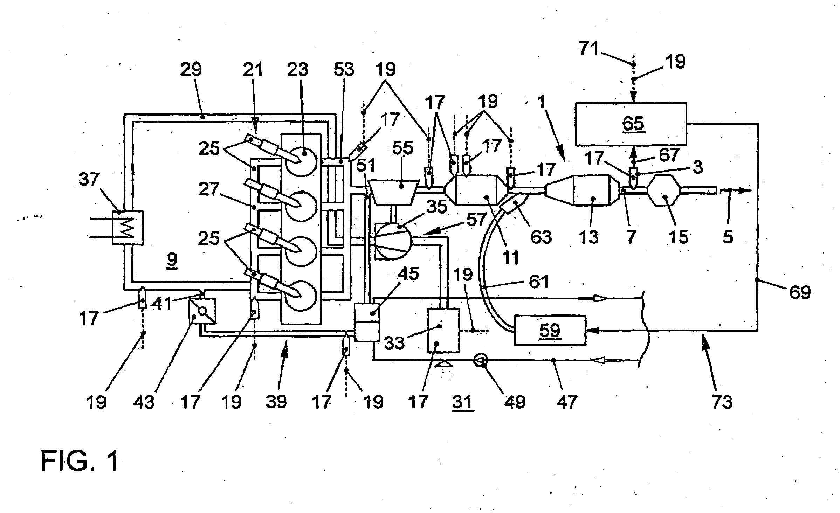

Figur 1 zeigt eine Abgasreinigungsvorrichtung 1 mit einer Sensorvorrichtung 3. Die Abgasreinigungsvorrichtung 1 dient zum Reinigen eines mittels eines Pfeils 5 symbolisierten Abgasstroms 7 eines Verbrennungsmotors 9. Die Abgasreinigungsvorrichtung 1 ist dem Verbrennungsmotor 9 nachgeschaltet. Zur Reinigung des Abgasstroms 7 weist die Abgasreinigungsvorrichtung 1 einen Dieselpartikelfilter 11, diesem nachgeschaltet einen SCR-Katalysator 13 und diesem nachgeschaltet einen Ammoniak (NH₃)-Sperrkatalysator 15 auf. Alternativ sind andere Anordnungen von Abgasreinigungskomponenten denkbar, beispielsweise zwei SCR Katalysatoren oder eine abweichende Reihenfolge. Zur Steuerung und/oder Regelung einer Gemischbildung des Verbrennungsmotors 9 und/oder einer Funktion der Abgasreinigungsvorrichtung 1 sind verschiedene Messstellen 17 vorgesehen. Die Sensorvorrichtung 3 ist dem SCR-Katalysator 13 nachgeschaltet und stellt eine der Messstellen 17 dar. Eine weitere, dem Dieselpartikelfilter 11 vorgeschaltete Messstelle 17 ist als λ-Sonde ausgelegt. Bei den übrigen Messstellen kann es sich beispielsweise um Temperatur, Luftmassen und/oder Drucksensoren handeln.

Eine in Figur 1 nicht näher dargestellte Gesamtsteuerung zur Steuerung und/oder Regelung der Abgasreinigungsvorrichtung 1 und/oder des Verbrennungsmotors 9 kann Messwerte der Messstellen 17 aufnehmen, was in Figur 1 schematisch mittels gepunkteter Linien 19 angedeutet ist.

Bei dem Verbrennungsmotor 9 kann es sich beispielsweise um einen Dieselmotor mit einer Commonrail 21 handeln. Der Commonrail 21 sind zum Einspritzen eines Kraftstoffs in Brennräume 23 des Verbrennungsmotors 9 insgesamt vier Commonrailinjektoren 25 zugeordnet. Zur Versorgung der Brennräume 23 mit Verbrennungsluft ist diesen ein Ansaugkrümmer 27 zugeordnet. Der Ansaugkrümmer 27 ist einem Ansaugkanal 29 nachgeschaltet. Der Ansaugkanal 29 weist eine lediglich mittels des Bezugszeichens 31 schematisch angedeuteten Lufteinlass 31, diesem nachgeschaltet einen Luftmassenmesser 33, diesem nachgeschaltet einen Verdichter 35, diesem nachgeschaltet einen Ladeluftkühler 37 auf. Der Ladeluftkühler 37 ist dem Ansaugkrümmer 27 vorgeschaltet. Der Verbrennungsmotor 9 weist eine Hochdruckabgasrückführung 39 auf, die zwischen dem Ladeluftkühler 37 und dem Ansaugkrümmer 27 in den Ansaugkanal 29 an einer Einmündungsstelle 41 in den Ansaugkanal 29 einmündet. Stromaufwärts der Einmündungsstelle 41 weist die Hochdruckabgasrückführung 39 ein Abgasrückführungsventil 43 auf. Mittels des Abgasrückführungsventils 43 kann ein zurückgeführter Massenstrom des Abgasstroms 7 in den Ansaugkanal 29 eingestellt werden. Stromabwärts des Abgasrückführungsventils 43 weist die Hochdruckabgasrückführung 39 einen Abgasrückführungskühler 45 auf. Der Abgasrückführungskühler 45 dient zum Kühlen des zurückgeführten Teilstroms des Abgasstroms 7 und ist zu diesem Zweck einem Niedertemperaturkreis 47, der eine Niedertemperaturpumpe 49 aufweist, zugeordnet. Bei dem Niedertemperaturkreis 47 kann es sich um einen üblicherweise vorhandenen Kühlkreis bzw. einen Teilkreis eines solchen, des Verbrennungsmotors 9, handeln. Der zurückgeführte Abgasteilstrom wird dem Abgasstrom 7 an einer Abzweigstelle 51 in die Hochdruckabgasrückführung 39 abgezweigt. Die Abzweigstelle 51 ist einem Auslasskrümmer 53 des Verbrennungsmotors 9 nachgeschaltet. Mittels des Auslasskrümmers 53 kann der Abgasstrom 7 aus den Brennräumen 23 des Verbrennungsmotors 9 abgeführt werden. Stromabwärts der Abzweigstelle 51 ist dem Auslasskrümmer 53 eine Turbine 55 nachgeschaltet. Mittels der Turbine 55 kann der Verdichter 35 des Ansaugkanals 29 mechanisch angetrieben werden. Die Turbine 55 und der Verdichter 35 bilden einen Turbolader 57. Stromabwärts des Turboladers 57 ist die Abgasreinigungsvorrichtung 1 zur Reinigung des Abgasstroms 7 angeordnet.

Die Abgasreinigungsvorrichtung 1 ist zur Reinigung des Abgasstroms 7 mittels einer selektiven katalytischen Reduktion ausgelegt. Dazu weist die Abgasreinigungsvorrichtung 1 eine Dosiervorrichtung 59 auf. Mittels der Dosiervorrichtung 59 kann ein Reaktionsmittel 61, beispielsweise ein Reduktionsmittel, insbesondere eine flüssige Harnstofflösung, dem Abgasstrom 7 zudosiert werden. Das Reaktionsmittel 61 enthält Ammoniak oder eine Vorstufe desselben, wobei stromaufwärts des SCR-Katalysators 13 im Ergebnis dem Abgasstrom 7 gasförmiger Ammoniak zudosierbar ist. Hierzu ist der Dosiervorrichtung 59 eine stromabwärts des Dieselpartikelfilters 11 und stromaufwärts des SCR-Katalysators 13 in den Abgasstrom 7 mündende Dosierdüse 63 zugeordnet. Stromabwärts des SCR-Katalysators 13 ist die Sensorvorrichtung 3 angeordnet.

Die Sensorvorrichtung 3 kann zum Detektieren einer Stickoxidkonzentration des Abgasstroms 7 ausgelegt sein. Darüber hinaus kann die Sensorvorrichtung 3 zur Detektion eventuell nicht umgesetzten Ammoniaks ausgelegt sein bzw. dazu eine Querempfindlichkeit aufweisen. Die Sensorvorrichtung 3 generiert ein eine Anwesenheit der Stickoxide und/oder des Ammoniaks in dem Abgasstrom 7 kennzeichnendes Messsignal 67. Das Messsignal 67 wird mittels einer Regelsteuereinheit 65 ausgewertet. In Abhängigkeit des Messsignals 67 generiert die Regelsteuereinheit 65 ein Stellsignal 69 zur Ansteuerung der Dosiervorrichtung 59, die wiederum über die Dosierdüse 63 eine entsprechende Menge des Reaktionsmittels 61 dem Abgasstrom 7 zudosiert. Mittels der gepunkteten Linie 19 ist angedeutet, dass die Regelsteuereinheit 65 mit der nicht mehr dargestellten übergeordneten Steuerregeleinheit in Verbindung steht und/oder Teil derselben ist, beispielsweise einen Sollwert für eine Stickoxid und/oder Ammoniakkonzentration stromabwärts des SCR-Katalysators 13 in dem Abgasstrom 7 verarbeitet. Mittels des Sollwerts 71 und des Messsignals 67 kann vorteilhaft ein geschlossener Regelkreis 73 verwirklicht werden. Vorteilhaft kann.mittels des Regelkreises 73 eine besonders genaue Zudosierung des Reaktionsmittels 61 erreicht werden, beispielsweise um möglichst niedrige Stickoxidwerte einzuhalten und um Ammoniakdurchbrüche, die trotz des nachgeschalteten Ammoniaksperrkatalysators 15 zu unerwünschten Emissionen führen würden, bestmöglich zu verhindern.

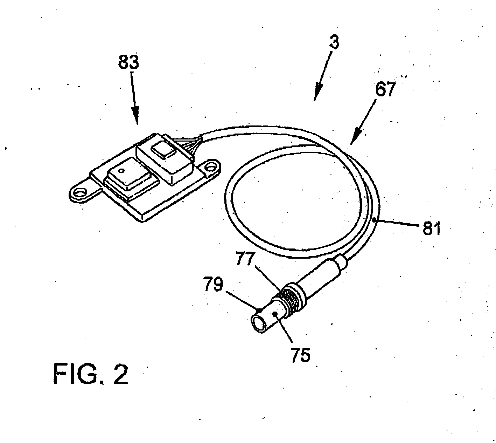

Vorteilhaft weist die Sensorvorrichtung 3 eine stabile Empfindlichkeit für Ammoniak auf, wobei vorteilhaft eine in dem Abgasstrom 7 vorhandene Ammoniakkonzentration möglichst präzise, insbesondere unabhängig von einer Temperatur und/oder Strömungsgeschwindigkeit des Abgasstroms 7, detektierbar ist. Eine solche vorteilhafte Sensorvorrichtung 3 wird im Folgenden anhand der Figuren 2 bis 6 näher beschrieben.

Figur 2 zeigt eine Sensorvorrichtung 3. Die Sensorvorrichtung 3 weist einen in einem Gehäuse 75 angeordneten, nicht näher dargestellten, Sensor auf. Das Gehäuse 75 kann mittels eines Gewindes 77 einer Abgasanlage so zugeordnet werden, dass ein Schutzrohr 79 des Gehäuses 75 zumindest teilweise in ein Messgas, beispielsweise in den Abgasstrom 7, eintauchen kann. Innerhalb des Schutzrohres 79 ist der nicht näher.dargestellte eigentliche Sensor der Sensorvorrichtung 3 angeordnet. Über ein Kabel 81 kann das Messsignal 67 einer Auswerteeinheit 83 zugeführt werden. Die Auswerteeinheit 83 kann beispielsweise der Regelsteuereinheit 65 zugeordnet werden oder Teil der Regelsteuereinheit 65 sein.

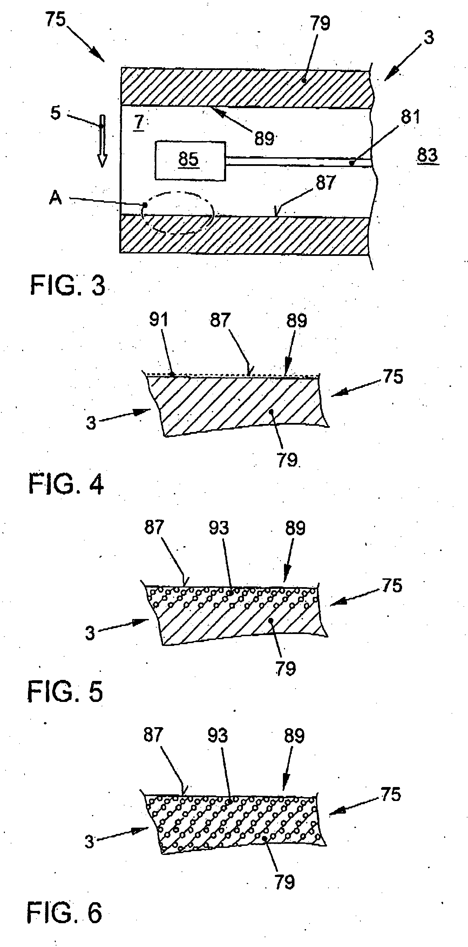

Die Figur 3 zeigt eine schematische Ansicht eines Teils eines Längsschnitts des Schutzrohres 79 des in Figur 2 dargestellten Gehäuses 75 der Sensorvorrichtung 3. Innerhalb des Schutzrohres 79 ist ein Sensor 85 der Sensorvorrichtung 3 angeordnet. Der Sensor 85 kommt mit dem Abgasstrom 7 zum ermitteln der Stickoxid und/oder Ammoniakkonzentration in Berührung. Eine Innenfläche 87 des Schutzrohres 79, die den Sensor 85 umgibt, kommt ebenfalls mit dem Abgasstrom 7 in Berührung. Um die Innenfläche 87 gegenüber dem Abgasstrom 7 möglichst inert, insbesondere katalytisch inaktiv, zu gestalten, weist die Innenfläche 87 eine Behandlung 89 auf.

Die Figur 4 zeigt ein in Figur 3 mit dem Buchstaben A gekennzeichnetes Detail der Innenfläche 87 des Schutzrohres 79 des Gehäuses 75 der Sensorvorrichtung 3. Gepunktet dargestellt ist die Behandlung 89 zu erkennen, die gemäß Figur 4 eine Oxidschicht 91 aufweist. Vorteilhaft ist die Oxidschicht 91 gegenüber Ammoniak nicht katalytisch aktiv. Die Oxidschicht 91 kann beispielsweise mittels Tempern des Gehäuses 75 in Anwesenheit einer sauerstoffhaltigen Atmosphäre oder mittels Nassbehandeln, beispielsweise mittels einer Behandlung mit Salpetersäure, insbesondere konzentrierter Salpetersäure, hergestellt werden.

Figur 5 zeigt das in Figur 4 dargestellte Detail, wobei jedoch im Unterschied die Behandlung 89 ein Katalysatorgift 93 aufweist. Das Katalysatorgift 93 ist mittels kleiner Kreise in Figur 5 symbolisiert. Es ist zu erkennen, dass nahe der Innenfläche 87 die Konzentration des Katalysatorgiftes 93 höher ist als weiter innen in einem Material des Gehäuses 75. Bei dem Material des Gehäuses 75 kann es sich beispielsweise um ein Metall handeln. Das Katalysatorgift 93 kann in die Innenfläche 87 des Schutzrohres 79 dotiert werden, beispielsweise mittels einer Diffusion oder einer Ionenimplantation. Vorteilhaft kann auch mittels des Katalysatorgiftes 93 eine katalytische Deaktivierung der Innenfläche 87 gegenüber Ammoniak verwirklicht werden.

Figur 6 zeigt das in Figur 5 gezeigte Detail, wobei im Unterschied das Katalysatorgift 93 in dem Material des Gehäuses 75 gleichverteilt ist. Hierzu kann das Material des Gehäuses 75 eine entsprechende Legierung aufweisen, der gleich bei einem Herstellungsprozess das Katalysatorgift 93 in einer entsprechenden Dosierung zugegeben wird.

Vorteilhaft kann die Abgasreinigungsvorrichtung 1 mit der Sensorvorrichtung 3 für Kraftfahrzeuge mit Dieselmotor und/oder magerbetriebenem Benzinmotor eingesetzt werden, wobei eine Abgasnachbehandlung mittels einer selektiven katalytischen Reduktion von in dem Abgasstrom 7 geführten Stickoxiden unter Zudosierung des Reaktionsmittels 61, beispielsweise der wässrigen Harnstofflösung, erfolgt. Der Sensor 85 der Sensorvorrichtung 3 weist neben einer Empfindlichkeit auf Stickoxide (NOₓ) auch eine Empfindlichkeit auf. Ammoniak (NH₃) auf. Bei einer Überdosierung des Reaktionsmittels 61 kann nach dem SCR-Katalysator 13 Ammoniak durchbrechen. Dies kann vorteilhaft mittels der Sensorvorrichtung 3 detektiert werden, so dass mittels der Regelsteuereinheit 65 sofort gegengesteuert werden kann. Vorteilhaft kann eine solche Regelung dank der Behandlung 89, also der katalytisch inaktiven Innenfläche 87 gegenüber Ammoniak, genauer erfolgen, wobei ein gegenüber Bedingungsschwankungen des Abgasstroms 7 robustes Messsignal 67 zum detektieren einer Anwesenheit des Ammoniaks in dem Abgasstrom 7 gewonnen werden. Das Messsignal 67 liefert also unabhängig von einer Temperatur und/oder Strömungsgeschwindigkeit und/oder weiteren Zustandsgrößen des Abgasstroms 7 ein gut auswertbares Messsignal 67 zum detektieren der Ammoniakdurchbrüche. Vorteilhaft ändert sich die Empfindlichkeit des Sensors 85 der Sensorvorrichtung 3 während des Betriebes des Kraftfahrzeuges gegenüber Ammoniak nicht oder nur sehr wenig, da die Innenfläche 87 mittels der Behandlung 89 gegenüber Ammoniak katalytisch inaktiv ist. Vorteilhaft kann daher an der Innenfläche 87 keine Reaktion von NH₃ zu N₂ und H₂O ablaufen. Vorteilhaft bleibt dadurch die Ammoniakkonzentration im Bereich des Sensors 85 des Abgasstroms 7 unverändert, kann also korrekt detektiert werden. Vorteilhaft findet auch keine Veränderung der ohnehin nicht vorhandenen katalytischen Aktivität der Innenfläche 87 während der Betriebszeit des Kraftfahrzeuges statt. Vorteilhaft kann eine stabile Steuerung und/oder Regelung der Dosierung des Reaktionsmittels 61 bzw. der Harnstofflösung ermöglicht werden.

Vorteilhaft kann dadurch auch ein vergleichsweise klein dimensionierter Katalysator für den SCR-Katalysator 13 vorgesehen werden. Der SCR-Katalysator 13 kann beispielsweise ein Volumen von weniger als 6, 4, 3 Litern, vorzugsweise nur 2 Litern, aufweisen. Vorteilhaft kann trotz des geringen Volumens des SCR-Katalysators 13 mittels der Regelsteuereinheit 65 ein Ammoniakschlupf verhindert oder zumindest auf ein Minimum reduziert werden.

Das Schutzrohr 79 der Sensorvorrichtung 3 wird vorteilhaft so behandelt, dass die Schutzrohroberfläche bzw. die Innenfläche 87 des Schutzrohres 69 nicht katalytisch aktiv ist und vorteilhaft diese auch während eines Betriebs des Kraftfahrzeuges nicht katalytisch aktiviert wird.

Dies kann auch vorteilhaft beispielsweise dadurch erreicht werden, dass das Schutzrohr 79 vor einem Verbau in dem Kraftfahrzeug bei einer hohen Temperatur, beispielsweise oberhalb von 800°C, einer sauerstoffhaltigen Atmosphäre ausgesetzt wird. Durch diesen Prozess bildet sich die Oxidschicht 91, die eine durchgängige Oxidschicht 91 bilden kann, die verhindert, dass Ammoniak an aktive Metalloberflächen gelangt, die eine Reaktion katalysieren können. Die Oxidschicht 91 ist während des Betriebs auch bei einer Beaufschlagung mit Fettgaskomponenten oder Ammoniak chemisch stabil. Alternativ und/oder zusätzlich kann die Oxidschicht 91 bzw. die Schutzschicht auch nasschemisch, z.B. durch eine Behandlung mit konzentrierter Salpetersäure erzeugt werden.

Zusammenfassend lässt sich eine Abgasvorrichtung 1 realisieren, die insgesamt weniger Bauraum benötigt, Kosten einspart und durch eine genauere Regelung der Dosierung des Reaktionsmittels 61 auch verbesserte Emissionswerte aufweist.

### Bezugszeichenliste

- 1: Abgasreinigungsvorrichtung
- 3: Sensorvorrichtung
- 5: Pfeil
- 7: Abgasstrom
- 9: Verbrennungsmotor
- 11: Dieselpartikelfilter
- 13: SCR-Katalysator
- 15: Ammoniak-Sperrkatalysator
- 17: Messstelle
- 19: Linie
- 21: Commonrail
- 23: Brennräume
- 25: Commonrailinjektoren
- 27: Ansaugkrümmer
- 29: Ansaugkanal
- 31: Lufteinlass
- 33: Luftmassenmesser
- 35: Verdichter
- 37: Ladeluftkühlung
- 39: Hochdruckabgasrückführung
- 41: Einmündungsstelle
- 43: Abgasrückführungsventil
- 45: Abgasrückfütirungskühler
- 47: Niedertemperaturkreis
- 49: Niedertemperaturpumpe
- 51: Abzweigstelle
- 53: Auslasskrümmer
- 55: Turbine
- 57: Turbolader
- 59: Dosiervorrichtung
- 61: Reaktionsmittel
- 63: Dosierdüse
- 65: Regelsteuereinheit
- 67: Messsignal
- 69: Stellsignal
- 71: Sollwert
- 73: Regelkreis
- 75: Gehäuse
- 77: Gewinde
- 79: Schutzrohr
- 81: Kabel
- 83: Auswerteeinhelt
- 85: Sensor
- 87: Innenfläche
- 89: Behandlung
- 91: Oxidschicht
- 93: Katalysatorgift

## Patentansprüche

1. Sensorvorrichtung zum Messen zumindest einer Gaskonzentration in einem Abgasstrom (7) eines Verbrennungsmotors (9), mit:
- einem für Stickoxide (NOₓ) und Ammoniak (NH₃) empfindlichen Sensor (85),
- einem Gehäuse (75) in dem der Sensor (85) aufgenommen ist,
**dadurch gekennzeichnet, dass** die dem Sensor (85) benachbarte Innenfläche (87) des Gehäuses (75) gegenüber Ammoniak im Wesentlichen katalytisch inaktiv ist oder mit einer Behandlung (89) in einen gegenüber Ammoniak im Wesentlichen katalytisch inaktiven Zustand versetzt ist.

2. Sensorvorrichtung nach dem vorhergehenden Anspruch, **dadurch gekennzeichnet, dass** das Material des Gehäuses (75) zumindest ein Metall oder eine Metalllegierung umfasst.

3. Sensorvorrichtung nach dem vorhergehenden Anspruch, **dadurch gekennzeichnet, dass** die Behandlung (89) die Bildung einer Oxidschicht (91) des Metalls umfasst.

4. Sensorvorrichtung nach dem vorhergehenden Anspruch, **dadurch gekennzeichnet, dass** die Oxidschicht (91) gegen den Fettgaskomponenten und den Ammoniak des Abgasstroms (7) chemisch stabil ist.

5. Sensorvorrichtung nach einem der vorhergehenden zwei Ansprüche, **dadurch gekennzeichnet, dass** die Oxidschicht (91) mittels Tempern des Gehäuses (75) bei hohen Temperaturen, insbesondere oberhalb von 800°C, und Aussetzen einer sauerstoffhaltigen Atmosphäre herstellbar ist.

6. Sensorvorrichtung nach einem der vorhergehenden drei Ansprüche, **dadurch gekennzeichnet, dass** die Oxidschicht (91) mittels Beaufschlagen mit Salpetersäure, insbesondere konzentrierter Salpetersäure, herstellbar oder hergestellt ist.

7. Sensorvorrichtung nach einem der vorhergehenden Ansprüche 2 bis 6, **dadurch gekennzeichnet, dass** das Metall des Gehäuses (75) einen austenetischen Edelstahl aufweist.

8. Sensorvorrichtung nach einem der vorhergehenden Ansprüche 2 bis 7, **dadurch gekennzeichnet, dass** das Metall des Gehäuses (75) eine Nickel(Ni)-Basis-Legierung aufweist.

9. Sensorvorrichtung nach einem der vorhergehenden Ansprüche, **dadurch gekennzeichnet, dass** die Behandlung (89) das Beifügen eines Katalysatorgiftes (93) umfasst.

10. Sensorvorrichtung nach dem vorhergehenden Anspruch, **dadurch gekennzeichnet, dass** das Katalysatorgift (93) Schwefel aufweist.

11. Sensorvorrichtung nach einem der vorhergehenden zwei Ansprüche, **dadurch gekennzeichnet, dass** das Katalysatorgift (93) mittels Diffusion beifügbar ist.

12. Abgasreinigungsvorrichtung (1) zur Reinigung eines Abgasstroms (7) eines Verbrennungsmotors (9) eines Kraftfahrzeuges mittels einer selektiven katalytischen Reduktion, mit:
- einem in den Abgasstrom (7) geschalteten SCR-Katalysator (13),
- einer dem SCR-Katalysator (13) vorgeschalteten Dosiervorrichtung (59) zum Dosieren eines Reaktionsmittels (61) in den Abgasstrom (7),
- einer dem SCR-Katalysator (13) und der Dosiervorrichtung (59) zugeordneten Sensorvorrichtung (3),
**dadurch gekennzeichnet, dass** die Sensorvorrichtung (3) nach einem der vorhergehenden Ansprüche ausgebildet ist.

13. Abgasreinigungsvorrichtung nach dem vorhergehenden Anspruch, **dadurch gekennzeichnet, dass** der SCR-Katalysator (13) ein Volumen von weniger als 6 Liter, insbesondere 4 oder 3 Liter, vorzugsweise ca. 2 Liter, aufweist.

14. Verfahren zum Herstellen einer Sensorvorrichtung (3) nach einem der vorhergehenden Ansprüche 1 bis 11, **gekennzeichnet durch**:
- Versehen der Innenfläche (87) des Gehäuses (75) mit der Behandlung (89), die gegenüber dem Ammoniak im Wesentlichen keine katalytische Aktivität aufweist.

15. Verfahren nach dem vorhergehenden Anspruch, **gekennzeichnet durch** zumindest einen Schritt der folgenden Gruppe:
- Oxidieren der Innenfläche (87),
- Oxidieren der Innenfläche (87) mittels Tempern der Innenfläche (87) in Anwesenheit einer sauerstoffhaltigen Atmosphäre,
- Oxidieren der Innenfläche (87) mittels Nassbehandeln,
- Nassbehandeln der Innenfläche (87) mittels Salpetersäure,
- Nassbehandeln der Innenfläche (87) mittels konzentrierter Salpetersäure,
- Beifügen eines Katalysatorgifts (93) zu der Innenfläche (87),
- Beifügen des Katalysatorgifts (93) mittels Dotieren,
- Beifügen des Katalysatorgifts (93) mittels Diffusion,
- Beifügen des Katalysatorgifts (93) mittels Ionenimplantation,
- Beifügen des Katalysatorgifts (93) in Form von Schwefel.

## Claims

1. Sensor device for measuring at least one gas concentration in an exhaust-gas stream (7) of an internal combustion engine (9), said sensor device having:
- a sensor (85) sensitive to nitrogen oxides (NOₓ) and ammonia (NH₃),
- a housing (75), in which the sensor (85) is accommodated,
**characterized in that** the inner surface (87), adjacent to the sensor (85), of the housing (75) is substantially catalytically inactive with respect to ammonia or is transferred by a treatment (89) into a state which is substantially catalytically inactive with respect to ammonia.

2. Sensor device according to the preceding claim, **characterized in that** the material of the housing (75) comprises at least one metal or a metal alloy.

3. Sensor device according to the preceding claim, **characterized in that** the treatment (89) comprises the formation of an oxide layer (91) of the metal.

4. Sensor device according to the preceding claim, **characterized in that** the oxide layer (91) is chemically stable against the rich gas components and the ammonia of the exhaust-gas stream (7).

5. Sensor device according to either of the preceding two claims, **characterized in that** the oxide layer (91) can be produced by means of a heat treatment of the housing (75) at high temperatures, in particular above 800°C, and exposure to an oxygen-containing atmosphere.

6. Sensor device according to one of the preceding three claims, **characterized in that** the oxide layer (91) can be produced or is produced by means of the application of nitric acid, in particular concentrated nitric acid.

7. Sensor device according to one of the preceding Claims 2 to 6, **characterized in that** the metal of the housing (75) comprises an austenitic high-grade steel.

8. Sensor device according to one of the preceding Claims 2 to 7, **characterized in that** the metal of the housing (75) comprises a nickel(Ni)-based alloy.

9. Sensor device according to one of the preceding claims, **characterized in that** the treatment (89) comprises the addition of a catalyst poison (93).

10. Sensor device according to the preceding claim, **characterized in that** the catalyst poison (93) comprises sulphur.

11. Sensor device according to either of the preceding two claims, **characterized in that** the catalyst poison (93) can be added by means of diffusion.

12. Exhaust-gas cleaning device (1) for cleaning an exhaust-gas stream (7) of an internal combustion engine (9) of a motor vehicle by means of selective catalytic reduction, said exhaust-gas cleaning device having:
- an SCR catalytic converter (13) connected in the exhaust-gas stream (7),
- a metering device (59), connected upstream of the SCR catalytic converter (13), for metering a reagent (61) into the exhaust-gas stream (7),
- a sensor device (3) assigned to the SCR catalytic converter (13) and to the metering device (59),
**characterized in that** the sensor device (3) is designed according to one of the preceding claims.

13. Exhaust-gas cleaning device according to the preceding claim, **characterized in that** the SCR catalytic converter (13) has a volume of less than 6 litres, in particular 4 or 3 litres, preferably approximately 2 litres.

14. Method for producing a sensor device (3) according to one of the preceding Claims 1 to 11, **characterized by**:
- providing the inner surface (87) of the housing (75) with the treatment (89), which has substantially no catalytic activity with respect to the ammonia.

15. Method according to the preceding claim, **characterized by** at least one step from the following group:
- oxidizing the inner surface (87),
- oxidizing the inner surface (87) by means of a heat treatment of the inner surface (87) in the presence of an oxygen-containing atmosphere,
- oxidizing the inner surface (87) by means of a wet treatment,
- wet-treating the inner surface (87) by means of nitric acid,
- wet-treating the inner surface (87) by means of concentrated nitric acid,
- adding a catalyst poison (93) to the inner surface (87),
- adding the catalyst poison (93) by means of doping,
- adding the catalyst poison (93) by means of diffusion,
- adding the catalyst poison (93) by means of ion implantation,
- adding the catalyst poison (93) in the form of sulphur.

## Revendications

1. Ensemble de capteur permettant de mesurer la concentration d'au moins un gaz dans un écoulement (7) de gaz d'échappement d'un moteur (9) à combustion interne, l'ensemble comprenant :
un capteur (85) sensible aux oxydes d'azote (NOₓ) et à l'ammoniac (NH₃) et
un boîtier (75) dans lequel le capteur (85) est logé,
**caractérisé en ce que**
la surface intérieure (87) du boîtier (75) voisine du capteur (85) est catalytiquement essentiellement inactive vis-à-vis de l'ammoniac ou est mise à l'aide d'un traitement (89) dans un état catalytiquement essentiellement inactif vis-à-vis de l'ammoniac.

2. Ensemble de capteur selon la revendication précédente, **caractérisé en ce que** le matériau du boîtier (75) comporte au moins un métal ou un alliage de métaux.

3. Ensemble de capteur selon la revendication précédente, **caractérisé en ce que** le traitement (89) comporte la formation d'une couche (91) d'oxyde du métal.

4. Ensemble de capteur selon la revendication précédente, **caractérisé en ce que** la couche (91) d'oxyde est chimiquement stable vis-à-vis des composants de gaz riche et de l'ammoniac de l'écoulement (7) de gaz d'échappement.

5. Ensemble de capteur selon l'une des deux revendications qui précèdent, **caractérisé en ce que** la couche (91) d'oxyde peut être formée par traitement du boîtier (75) à haute température, en particulier au-dessus de 800°C, et exposition à une atmosphère contenant de l'oxygène.

6. Ensemble de capteur selon l'une des trois revendications qui précèdent, **caractérisé en ce que** la couche (91) d'oxyde peut être formée ou est formée par application d'acide nitrique et en particulier d'acide nitrique concentré.

7. Ensemble de capteur selon l'une des revendications 2 à 6 qui précèdent, **caractérisé en ce que** le métal du boîtier (75) présente un acier allié austénitique.

8. Ensemble de capteur selon l'une des revendications 2 à 7 qui précèdent, **caractérisé en ce que** le métal du boîtier (75) présente un alliage à base de nickel (Ni).

9. Ensemble de capteur selon l'une des revendications qui précèdent, **caractérisé en ce que** le traitement (89) comporte l'adjonction d'un poison (93) de catalyseur.

10. Ensemble de capteur selon la revendication précédente, **caractérisé en ce que** le poison (93) de catalyseur présente du soufre.

11. Ensemble de capteur selon l'une des revendications qui précèdent, **caractérisé en ce que** le poison (93) de catalyseur peut être ajouté par diffusion.

12. Ensemble (1) d'épuration des gaz d'échappement destiné à épurer l'écoulement (7) de gaz d'échappement d'un moteur (9) à combustion interne d'un véhicule automobile par réduction catalytique sélective l'ensemble présentant :
un catalyseur SCR (13) raccordé dans l'écoulement (7) de gaz d'échappement,
un ensemble de dosage (59) raccordé en amont du catalyseur SCR (13) et permettant de doser un réactif (61) dans l'écoulement (7) de gaz d'échappement et
un ensemble de capteur (3) associé au catalyseur SCR (13) et à l'ensemble de dosage (59),
**caractérisé en ce que**
l'ensemble de capteur (3) est configuré selon l'une des revendications précédentes.

13. Ensemble d'épuration des gaz d'échappement selon la revendication précédente, **caractérisé en ce que** le catalyseur SCR (13) présente un volume inférieur à 6 litres, en particulier à 4 ou 3 litres, et est de préférence d'environ 2 litres.

14. Procédé de fabrication d'un ensemble de capteur (3) selon l'une des revendications 1 à 11 qui précèdent, **caractérisé par** l'étape qui consiste à appliquer sur la surface intérieure (87) du boîtier (75) un traitement (89) qui ne présente essentiellement aucune activité catalytique vis-à-vis de l'ammoniac.

15. Procédé selon la revendication précédente, **caractérisé par** au moins une étape de l'ensemble suivait
oxydation de la surface intérieure (87),
oxydation de la surface intérieure (87) par traitement thermique de la surface intérieure (87) en présence d'une atmosphère contenant de l'oxygène,
oxydation de la surface intérieure (87) par traitement en conditions humides,
traitement en conditions humides de la surface intérieure (87) par acide nitrique, traitement en conditions humides de la surface intérieure (87) par acide nitrique concentré,
adjonction d'un poison (93) de catalyseur à la surface intérieure (87),
adjonction du poison (93) du catalyseur par dopage,
adjonction du poison (93) du catalyseur par diffusion,
adjonction du poison (93) du catalyseur par implantation d'ions et
adjonction du poison (93) du catalyseur sous la forme de soufre.
